# EUROPEAN PATENT SPECIFICATION

(11) **EP 1 804 743 B1**
(45) Date of publication and mention of the grant of the patent: **20.03.2013**
(21) Application number: 04768795.9
(22) Date of filing: 08.10.2004
(51) Int. Cl.: A61F 5/44

(54) **URINE COLLECTION BAG**
HARNSAMMELBEUTEL
SAC COLLECTEUR D'URINE

(30) Priority: 13.11.2003 GB 0326438; 02.12.2003 GB 0327982; 05.03.2004 GB 0405002
(43) Date of publication of application: 11.07.2007
(73) Proprietor: Manojlovic, Nada Dorothy, Thorner, Leeds LS14 3EG (GB)
(72) Inventor: Manojlovic, Nada Dorothy, Thorner, Leeds LS14 3EG (GB)
(74) Representative: Sherrard-Smith, Hugh
(86) International application number: PCT/GB2004/004261
(87) International publication number: WO 2005/053583

(56) References cited:
- WO-A-00/33773
- WO-A-91/00074
- WO-A-03/032876
- DE-U- 9 004 582
- FR-A- 2 127 704
- GB-A- 2 295 766
- US-A- 4 511 358
- US-A- 4 581 763
- US-A- 5 032 118

## Description

The present invention relates to an undergarment having a pocket in which a urine collection bag is arranged, in use, to be held by the pocket.

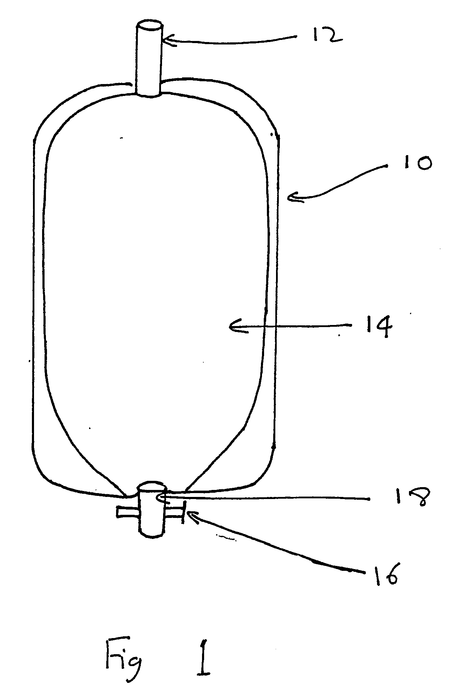

Urine collection bags currently available are generally rectangular in shape with an inlet pipe at a top end and an outlet pipe at a bottom end. Such a conventional urine collection bag can be seen in Figure 1.

A conventional urine collection bag 10, as shown in Figure 1, is connected to an in dwelling catheter, either urethral or supra pubic, via inlet pipe 12. Urine enters the urine collection bag 10 from the catheter and is collected and stored in a collection chamber 14. Outlet pipe 18 is used to drain urine from the collection chamber 14. The escape of urine from the urine collection bag is prevented by outlet valve 16. When collection chamber 14 is full, valve 16 is opened to allow urine to drain from collection chamber 14 into outlet pipe 18. Outlet pipe 18 may be positioned over a toilet bowl such that the urine may be safely and hygienically disposed off.

Conventional urine collection bags, such as that shown in Figure 1, are held in place in the groin area of a user in a number of ways. The urine collection bag may be strapped to a users leg. The urine collection bag may be held in a leg bandage comprising a pocket to hold said urine bag. A user may wear a suspender belt arrangement that holds the urine collection bag close to his or her leg. Finally, a user may wear a belly bag device into which the urine collection bag may be placed.

Known urine collection bags and holding methods have many disadvantages.

Known bags are bulky and uncomfortable to wear. Known bags fill the collection chamber from bottom to top, as the urine drains into the collection chamber, the urine collection bag is deformed from its original flat shape. The urine produces a bulge in the bag. This bulge is uncomfortable and can become visible beneath a users clothing. As the bag fills there is nothing to stop the urine moving inside the bag. As the user moves the urine can slosh from side to side or up and down, again this is uncomfortable to the user. When the bag is full or nearly full, the weight of the urine contained in the collection chamber can cause the top of the bag to lean forwards. This is uncomfortable and can cause the bag to become more pronounced beneath a user's clothing.

W0 00/33773 discloses a serpentine shaped reservoir that is sewn into the front panel of a pair of briefs. WO 03/032876 discloses a waste protection chamber that is secured in the crotch region of a wearer by a strap.

Known holding methods are uncomfortable and do not allow a user easy access to the outlet valve and outlet pipe. A large amount of time and effort is required to gain access to the outlet valve and outlet pipe so that the bag may be emptied.

It is an object of the present invention to attempt to overcome at le a s t some of the above or other disadvantageous.

According to the present invention there is provided an apparatus as set forth in the appended claims.

The urine collection bag pocket may comprise at least one outlet hole whereby an outlet pipe of the urine collection bag may be passed through said outlet hole such that a user may empty urine from the urine collection bag without taking the undergarment off or removing the urine collection bag from the urine collection bag pocket. The undergarment may include a plurality of outlet holes which may be at the same elevation. The undergarment may include a plurality of outlet holes including one to each side of the front centre of the undergarment. The plurality of holes may be at different elevations.

At least one outlet hole may be adjacent to the edge of the undergarment.

The undergarment may be made of a seam-free fabric which may be polyaminde elastine.

The undergarment may be a pair of underpants.

The undergarment may include an extended gusset greater than 5cm and preferably in the region of 10cm wide.

The urine collection bag pocket may be located at the front of the underpants.

The undergarment may be a pair of boxer shorts.

An outlet pipe of the urine collection bag may extend through an outlet hole in the pocket.

A user may select one of a plurality of outlets of the undergarment through which urine is arranged to be discharged.

A user may put the undergarment on and then locate the bag in the pocket.

A urine collection chamber may be shaped to fit into a groin region of the user.

A urine collection chamber may be of greater width at an upper region than it is at a lower region and may decrease in width continuously from an upper region to a lower region and may be triangular in shape.

An inlet pipe of the bag may be positioned centrally to an upper portion of the urine collection bag.

An outlet pipe of the bag may be positioned on a lower portion of the urine collection bag.

The outlet pipe may comprise an outlet valve to control the flow of liquid from the urine collection chamber into said outlet pipe.

The chamber of the bag may be impervious to water.

The urine collection chamber may comprise a plurality of urine collection compartments and said urine collection compartments may be separated by at least one inner wall and said inner wall or walls may be arranged to be substantially horizontal when the urine collection bag is placed in the groin region of the user.

Said urine collection compartments may be connected to each other, such that, in use, urine drains from the inlet pipe through the upper compartment to a lower compartment or compartments and may be arranged to drain downwardly in a line through at least two collection compartments.

Said urine collection compartments may be connected to each other and the inlet pipe by a plurality of drainage channels and said drainage channels may be arranged to be substantially vertical when the urine collection bag is placed in the groin region of the user or may be arranged to be substantially at an angle to the vertical when the urine collection bag is placed in the groin region of the user.

A first urine collection compartment may be connected to the outlet pipe to allow urine to drain from the urine collection chamber when the outlet valve is opened.

The inner walls may be formed by heat-sealing strips of sidewalls of the urine collection chamber together.

Divider sections may be provided in the urine collection chamber to form the inner wall or walls.

The urine collection bag may comprise a urine collection chamber, an inlet pipe arranged, in use, to connect a catheter to the chamber, an outlet pipe connected to the chamber to enable liquid to drain from the chamber, the chamber further including at least one wall extending at least partially across the chamber, for instance being spaced from the top and bottom of the chamber, and being arranged, in use, to restrict the flow of urine, for instance upwardly, past the or each wall. The wall may be connected to at least one side of the bag and the wall may be connected to both sides of the bag and may restrict movement of the sides to which it is connected away from each other as the bag fills with urine.

The urine collection bag may comprise a urine collection chamber, an inlet pipe arranged to connect to a catheter at a first end and the urine collection chamber at a second end, an outlet pipe connected to the urine collection chamber to enable liquid to drain from the urine collection chamber, the chamber further including a connection between opposed outer walls of the urine collection chamber arranged to restrict deformation of the urine collection chamber when urine drains from the inlet pipe into the urine collection chamber. Said connection may include at least one tie extending between the opposed outer walls and the tie or ties may be heat sealed to the collection chamber. The tie or ties may comprise walls.

The urine collection bag may comprise a urine collection chamber, an inlet pipe arranged to connect to a catheter at a first end and the urine collection chamber at a second end, an outlet pipe connected to the urine collection chamber to enable liquid to drain from the urine collection chamber, wherein said chamber is of a greater width in a first region than it is at a second region. The first region may be an upper region and the second region may be a lower region.

Said urine collection chamber may decrease in width continuously from the first region to the second region.

Preferred features of the invention will be apparent from the dependent claims, and the description which follows.

For a better understanding of the invention, and to show how embodiments of the same may be carried into effect, reference will now be made, by way of example, to the accompanying diagrammatic drawings in which:
Figure 1 is a side view of a known urine collection bag.
Figures 2 and 2A are side and perspective views of a preferred urine collection bag.
Figures 3 and 3A are a cut away side view and a perspective view of a preferred urine collection bag.
Figures 4, 4A, 4B and 4C are front views of an undergarment into which a preferred urine collection bag may be placed.
Figure 5 is a front view of an undergarment not in accordance with the present invention into which a preferred urine collection bag may be placed.

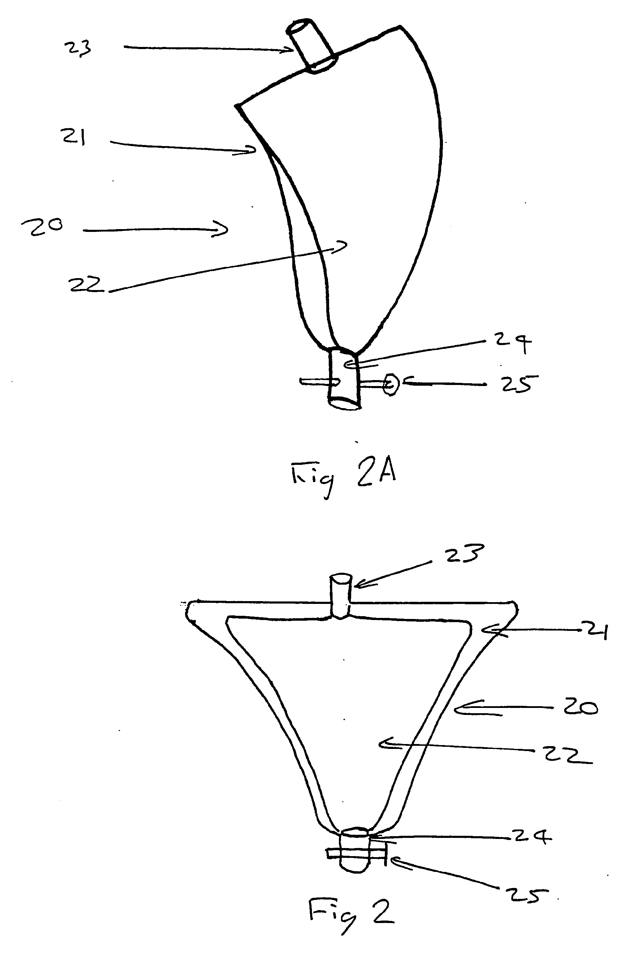

Referring to Figures 2 and 2A, a urine collection bag 20 according to a preferred embodiment of the present invention comprises an outer layer 21 forming a urine collection chamber 22, an inlet pipe 23, an outlet pipe 24, and an outlet valve 25.

The outer layer 21, which surrounds and forms the urine collection chamber 22, is triangular in shape. The triangular shape allows the urine collection bag 20 to comfortably and discretely fit in the groin area of a user's body.

The outer layer 21 is made from a waterproof material, for example a plastic such as polypropylene. To form the urine collection chamber 22, two sheets of shaped waterproof material are placed adjacent to each other and heat-sealed around the edge. This forms a watertight seal around the urine collection chamber 22 enabling urine to be collected in the urine collection chamber 22 without leakage.

In a preferred embodiment of the present invention, the outer layer 21 is covered with an outer cotton layer for the comfort of a user.

The inlet pipe 23 is provided towards a top end of the urine collection chamber 22. In use, inlet pipe 23 will be connected to a standard urethral or supra pubic catheter (not shown). The inlet pipe 23 allows urine to drain from the catheter into the urine collection chamber 22.

When the urine collection chamber 22 becomes full, a user may empty the chamber by opening outlet valve 25. This allows the urine to drain out of the urine collection chamber 22 via outlet pipe 24. Outlet pipe 24 is provided towards a bottom end of the urine collection chamber 22. Outlet valve 25 may be of a conventional type such as a push valve or a flip flow valve. The person skilled in the art would be familiar with all conventional valves and as such a detailed description of the outlet valve 25 is not disclosed herein. In this way emptying of the bag can be performed without any additional steps are required when a user goes to the urinal.

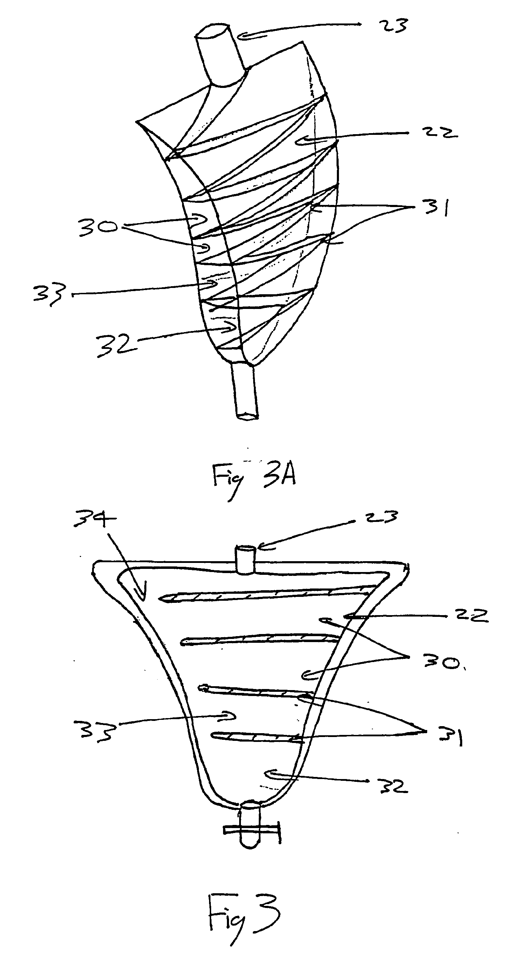

Referring to Figure 3 a cut away side view of a preferred urine collection bag is shown. Figures 3 and 3A show the urine collection chamber 22 of a preferred embodiment of the present invention. The urine collection chamber comprises a plurality of compartments 30 separated by walls 31. Walls 31 are preferably formed by heat-sealing sections of the two outer layer sections 21 in strips across the urine collection chamber 22. However, the skilled person would be aware of other methods of creating compartments 30, for example, walls 31 may be provided by introducing divider sections into the urine collection chamber 22.

The compartments 30 are all interconnected by a drainage channel 34. The drainage channel 34 allows urine to drain from the inlet pipe 23 into a lowest compartment that is not already full. The preferred embodiment shown in Figure 3 only shows one drainage channel 34 interconnecting the compartments 30, it will, however, be apparent to the person skilled in the art that a plurality of drainage channels may be provided between compartments 30 to aide in the even filling of the compartments 30.

The compartments 30 are provided in the urine collection chamber 22 such that the expansion of the urine collection bag 20 is controlled. Urine drains from a catheter, down the inlet pipe 23, into the urine collection chamber 22, down the drainage channel 34 and into the first compartment 32. As compartment 32 fills with urine the outer layer 21 will expand slightly and a bulge will form in the urine collection bag 20. The bulge is restricted as the walls act to restrict the expansion between opposed sides of the bag. When the first compartment 32 is full, urine will begin to drain into the second compartment 33. The outer layer of this second compartment will expand in the same way as the first compartment 32.

The provision of a plurality of compartments 30 within the urine collection chamber 22 allows the outer layer 21 to expand in small discrete amounts as the urine collection chamber fills. As the urine collection chamber becomes full the urine collection bag will deform discretely and evenly. The walls 31 add strength to the urine collection bag 20 and support the weight of the urine contained in the urine collection chamber 22. Additionally, the provision of chambers 30 restricts the movement of urine in the urine collection chamber 22. Urine within the urine collection chamber will be unable to slosh from side to side or up and down when a user moves.

The preferred urine collection bag 20, as shown in Figure 3, is provided with horizontal walls 31 which create horizontal compartments 30. The walls may be provided vertically or at an angle to the horizontal.

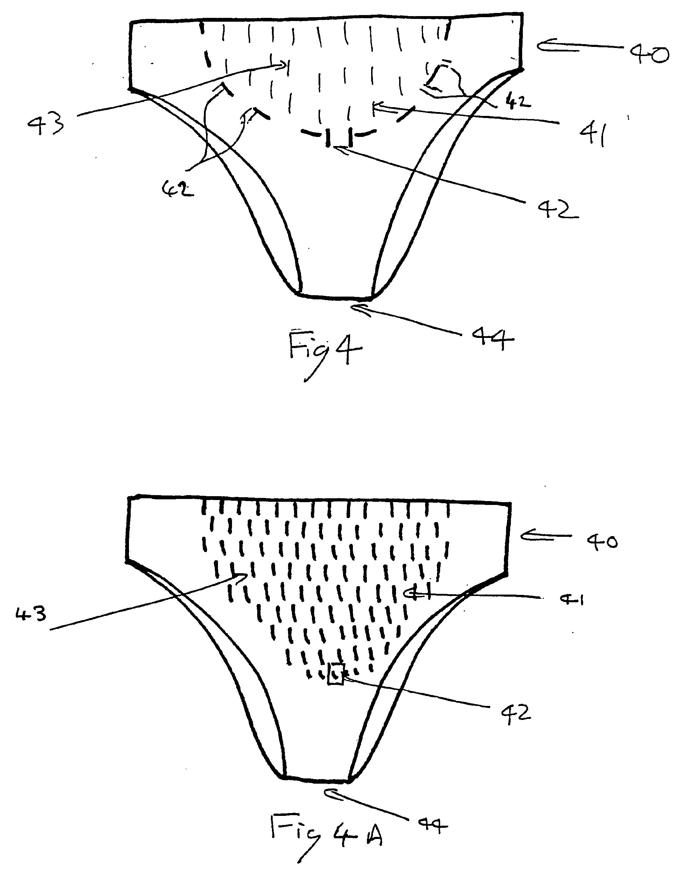

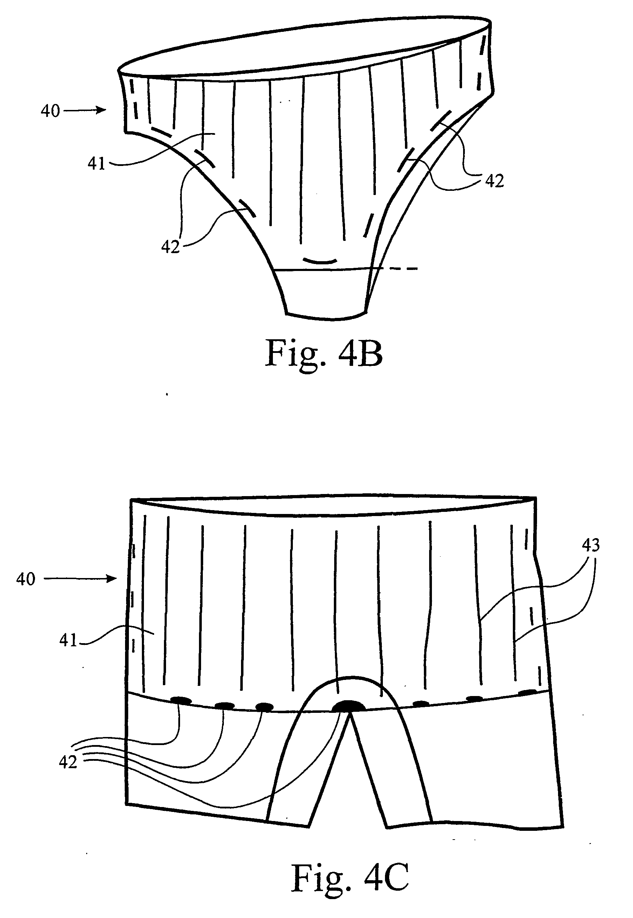

Referring now to Figures 4, 4A, 4B and 4C, an undergarment 40 in accordance with a preferred embodiment of the present invention comprises a pocket 41 into which a urine collection bag may be placed.

The undergarment 40 is in the form of a conventional pair of underpants or boxer shorts as shown in Figure 4C and will be worn as such by a user. The pocket 41 further comprises an opening or openings 42. One opening 42 is located in a central region of the undergarment 40 towards the bottom of the pocket 41. When a user places the urine collection bag into the pocket 41, an outlet pipe of the urine collection bag is pushed through an opening 42. The opening is selected in accordance with the preferred location for a user. Some may prefer to use the lower or central opening. Others may prefer an offset opening to one side or another. When the urine collection bag becomes full, a user may easily and conveniently reach the outlet pipe of the urine collection bag such that he or she may quickly and discretely empty the urine from the urine collection bag.

The undergarment 40 may be made from any material suitable for conventional underwear however, a preferred embodiment of the present invention is made from an elastic seam-free material such as polyamide elastine. The elasticity of the material allows the undergarment 40, and in particular the pocket 41, to stretch as the urine collection bag fills and expands. This increases comfort for a user. The undergarment 40 is provided seam-free such that as the pocket expands to accommodate the expanding urine collection bag, there are no seams that may dig into the skin of a user causing discomfort.

The pocket 41 of the undergarment 40 may be provided with ribbing 43. This increases the strength of the pocket 41 such that the urine collection bag may be better supported. It also adds comfort for a user.

The undergarment 40 may be provided with a gusset 44 of width approximately 10cm. This gusset size is greater than that found in conventional underpants. This increased gusset width allows the undergarment 40 to comfortably fit an incontinence pad. Most catheter users wear incontinence pads for protection against leaks. The greater gusset width allows a user to more comfortable and discretely wear an incontinence pad.

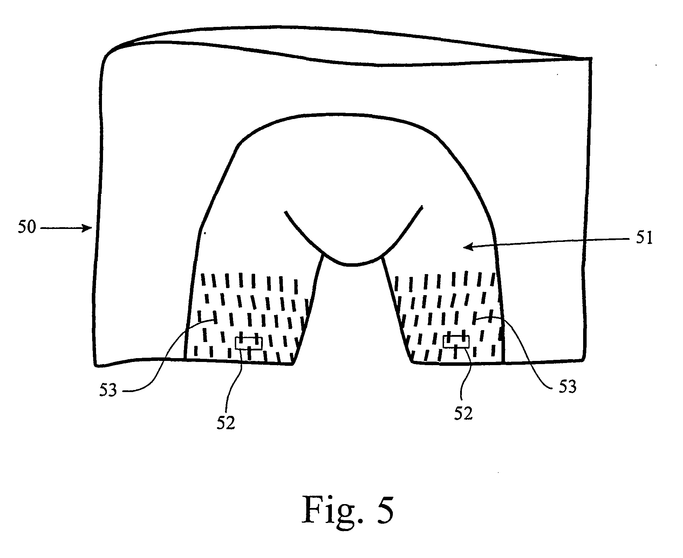

Referring to Figure 5, which is not in accordance with the present invention, and undergarment into which a urine collection bag may be placed is shown. This is in the form of a pair of boxer shorts 50. The boxer shorts 50 are provided with a pocket 51 on the inside leg of the garment. A user may discretely place a urine collection bag into the pocket 51. The pocket 51 is provided with an opening 52 that allows a user to conveniently reach an outlet pipe of the urine collection bag. This is described above in relation to the first embodiment of an undergarment.

Boxer shorts 50 can be made from any material suitable for conventional underwear however, a preferred embodiment is made from an elastic seam-free material as outlined above in relation to the first embodiment.

The pocket 51 of the boxer shorts 50 may be provided with ribbing 53. This increases the strength of the pocket 51 such that the urine collection bag may be better supported, it also adds to the comfort of a user.

Accordingly, it can be seen that the urine collection bag of the present invention is easier, more discrete and comfier to use than conventional urine collection bags. In addition the undergarment of the present invention provides a user with a more comfortable and discrete way of wearing a urine collection bag.

## Claims

1. An undergarment (40) comprising a urine collection bag pocket is adapted to hold a urine collection bag (20),
**characterised in that** said urine collection bag pocket (41)is arranged in use, to be located in the region of a user's groin.

2. An undergarment as claimed in claim 1 in which the pocket is shaped to conform to the shape of a user's groin.

3. An undergarment as claimed in claim 1 and 2 including a urine collection bag shaped to fit into a groin region of a user and located in the pocket.

4. An undergarment as claimed in claim 1, 2 or 3 wherein said urine collection bag pocket comprises at least one outlet hole whereby an outlet pipe of the urine collection bag may be passed through said outlet hole such that a user may empty urine from the urine collection bag without taking the undergarment off or removing the urine collection bag from the urine collection bag pocket.

5. An undergarment as claimed in any preceding claim including a plurality of outlet holes including one to each side of the front centre of the undergarment.

6. An undergarment as claimed in claims 4 or 5 including at least one outlet hole adjacent to the edge of the undergarment.

7. An undergarment as claimed in any of claims 1 to 5, wherein the urine collection bag pocket comprises ribbing to add support to the urine collection bag pocket and comfort for the user.

8. An undergarment as claimed in claim 7, wherein the undergarment is a pair of underpants and the underpants comprise an extended gusset to accommodate an incontinence pad.

9. An undergarment as claimed in any preceding claim including a urine collection bag located at least partially in the pocket.

10. An undergarment as claimed in claim 9 in which the urine collection bag comprises a urine collection chamber (22), an inlet pipe (23) arranged to connect to a catheter at a first end and the urine collection chamber at a second end, an outlet pipe (24) connected to the urine collection chamber to enable liquid to drain from the urine collection chamber, wherein said urine collection chamber is shaped such that it conforms to the shape of a users body.

11. An undergarment as claimed in claim 10 in which the urine collection bag as claimed in claim 11, wherein said urine collection chamber is shaped to fit into a groin region of the user.

12. An undergarment as claimed in claim 10 or 11 in which th e collection chamber decreases in width continuously from an upper region to a lower region and is triangular in shape wherein said triangular shape comprises two concave edges adjacent to each other such that the sidewalls conform to the inner thighs of the user.

13. An undergarment as claimed in claim 10, 11 or 12, wherein the inlet pipe is positioned centrally to an upper portion of the urine collection bag.

## Patentansprüche

1. Unterbekleidung (40) mit einer Tasche für Urinsammelbeutel, die zum Aufnehmen eines Urinsammelbeutels (20) ausgebildet ist,
**dadurch gekennzeichnet, dass** die Tasche für Urinsammelbeutel (41) bei Gebrauch in dem Bereich der Leistenbeuge des Benutzers positioniert ist.

2. Unterbekleidung nach Anspruch 1, wobei die Tasche derart geformt ist, dass sie sich der Form der Leistenbeuge eines Benutzers anpasst.

3. Unterbekleidung nach Anspruch 1 und 2 mit einem Urinsammelbeutel, der so geformt ist, dass er in den Bereich der Leistenbeuge eines Benutzers passt und in der Tasche positioniert ist.

4. Unterbekleidung nach Anspruch 1, 2 oder 3, wobei die Tasche für Urinsammelbeutel mindestens eine Auslassöffnung enthält, wobei ein Ableitungsrohr des Urinsammelbeutels durch die Auslassöffnung derart hindurchgeführt werden kann, dass ein Benutzer Urin aus dem Urinsammelbeutel entleeren kann, ohne die Unterbekleidung abzulegen oder den Urinsammelbeutel aus der Tasche für Urinsammelbeutel herauszunehmen.

5. Unterbekleidung nach einer der vorhergehenden Ansprüche, die mehrere Auslassöffnungen enthält, einschließlich einer an jeder Seite von dem vorderen Mittelteil der Unterbekleidung.

6. Unterbekleidung nach Anspruch 4 oder 5, die mindestens eine Auslassöffnung angrenzend an den Saum der Unterbekleidung enthält.

7. Unterbekleidung nach einem der Ansprüche 1 bis 5, wobei die Tasche für Urinsammelbeutel eine Rippung zur Verstärkung der Tasche für Urinsammelbeutel und den Komfort des Benutzers aufweist.

8. Unterbekleidung nach Anspruch 7, wobei die Unterbekleidung eine Unterhose ist und die Unterhose einen verbreiterten Zwickel zum Unterbringen einer Inkontinenzbinde aufweist.

9. Unterbekleidung nach einem der vorhergehenden Ansprüche mit einem Urinsammelbeutel, der mindestens teilweise in der Tasche positioniert ist.

10. Unterbekleidung nach Anspruch 9, in welcher der Urinsammelbeutel eine Urinsammelkammer (22), ein Einleitungsrohr (23), das zum Verbinden mit einem Katheter an einem ersten Ende und der Urinsammelkammer an einem zweiten Ende eingerichtet ist, und ein Ableitungsrohr (24) aufweist, das mit der Urinsammelkammer verbunden ist, um es zu ermöglichen, Flüssigkeit aus der Urinsammelkammer abzulassen, wobei die Urinsammelkammer derart geformt ist, dass sie sich der Form des Körpers von einem Benutzer anpasst.

11. Unterbekleidung nach Anspruch 10, die den Urinsammelbeutel nach Anspruch 1 enthält, wobei die Urinsammelkammer derart geformt ist, dass sie in den Bereich einer Leistenbeuge des Benutzers passt.

12. Unterbekleidung nach Anspruch 10 oder 11, in welcher die Sammelkammer kontinuierlich von einem oberen Bereich zu einem unteren Bereich in der Breite abnimmt und in der Form eines Dreiecks ist, wobei die dreieckige Form zwei gewölbte Ränder derart nebeneinanderliegend aufweist, dass die Seitenwände sich dem Innenbereich der Oberschenkel des Benutzers anpassen.

13. Unterbekleidung nach Anspruch 10, 11, oder 12, wobei das Einleitungsrohr mittig zu einem oberen Abschnitt des Urinsammelbeutels positioniert ist.

## Revendications

1. Sous-vêtement (40), comprenant une poche de sac collecteur d'urine, conçu pour contenir un sac collecteur d'urine (20),
**caractérisé en ce que** ladite poche de sac collecteur d'urine (41) est conçue de manière à être située, lors de l'utilisation, dans la région de l'aine de l'utilisateur.

2. Sous-vêtement selon la revendication 1, dans lequel la poche est configurée de manière à épouser la forme de l'aine d'un utilisateur.

3. Sous-vêtement selon la revendication 1 et 2, comprenant un sac collecteur d'urine qui est configuré de manière à s'agencer dans une région de l'aine d'un utilisateur et qui est situé dans la poche.

4. Sous-vêtement selon la revendication 1, 2 ou 3, dans lequel ladite poche de sac collecteur d'urine comporte au moins un trou de sortie, dans lequel un tube de sortie du sac collecteur d'urine peut être passé à travers ledit trou de sortie, de telle sorte qu'un utilisateur puisse vidanger l'urine hors du sac collecteur d'urine sans enlever le sous-vêtement, ou puisse enlever le sac collecteur d'urine de la poche de sac collecteur d'urine.

5. Sous-vêtement selon l'une quelconque des revendications précédentes, comprenant une pluralité de trous de sortie, y compris un de chaque côté de la région centrale avant du sous-vêtement.

6. Sous-vêtement selon la revendication 4 ou 5, comprenant au moins un trou de sortie situé à proximité du bord du sous-vêtement.

7. Sous-vêtement selon l'une quelconque des revendications 1 à 5, dans lequel la poche de sac collecteur d'urine comporte un nervurage pour renforcer le support de la poche de sac collecteur d'urine et pour augmenter le confort de l'utilisateur.

8. Sous-vêtement selon la revendication 7, dans lequel le sous-vêtement est un slip, et le slip comprend un soufflet étendu pour recevoir un tampon d'incontinence.

9. Sous-vêtement selon l'une quelconque des revendications précédentes, comprenant un sac collecteur d'urine qui est situé au moins partiellement dans la poche.

10. Sous-vêtement selon la revendication 9, dans lequel le sac collecteur d'urine comprend une chambre de collecte d'urine (22), un tube d'entrée (23) conçu pour être connecté à un cathéter à une première extrémité et à la chambre de collecte d'urine à une deuxième extrémité, un tube de sortie (24) connecté à la chambre de collecte d'urine afin de permettre le drainage du liquide hors de la chambre de collecte d'urine, dans lequel ladite chambre de collecte d'urine est configurée de telle sorte qu'elle épouse la forme du corps de l'utilisateur.

11. Sous-vêtement selon la revendication 10 équipé du sac collecteur d'urine selon la revendication 1, dans lequel ladite chambre de collecte d'urine est configurée de manière à s'adapter à la région de l'aine de l'utilisateur.

12. Sous-vêtement selon la revendication 10 ou 11, dans lequel la largeur de la chambre de collecte diminue de façon continue à partir d'une région supérieure jusqu'à une région inférieure, et est de forme triangulaire, dans lequel ladite forme triangulaire présente deux bords concaves adjacents l'un à l'autre de telle sorte que les parois latérales épousent l'intérieur des cuisses de l'utilisateur.

13. Sous-vêtement selon la revendication 10, 11 ou 12, dans lequel le tube d'entrée est positionné de façon centrale à une partie supérieure du sac collecteur d'urine.
